# EUROPEAN PATENT APPLICATION

(11) **EP 4 389 095 A1**
(43) Date of publication of application: **26.06.2024**
(21) Application number: 22216023.6
(22) Date of filing: 22.12.2022
(51) Int. Cl.: A61F 13/494

(54) **ABSORBENT ARTICLE WITH DOUBLE-LAYER CUFF**

(71) Applicant: Ontex BV, 9255 Buggenhout (BE); Ontex Group NV, 9320 Erembodegem (BE)
(72) Inventor: Lambertz, Christina, 56566 Neuwied (DE); WEBER, Ainas, 53474 Bad Neuenahr-Ahrweiler (DE)
(74) Representative: Macchetta, Andrea

(57) **Abstract**

The present disclosure relates to a disposable absorbent chassis having a liquid-pervious topsheet, a backsheet, and a liquid absorbent core disposed between the liquid-pervious topsheet and the backsheet. The chassis has a pair of longitudinal standing gathers and/or at least one waist barrier and a pair of elasticized outer leg barriers disposed on either side of the longitudinal central axis. Each of the longitudinal standing gathers and/or each of the at least one waist barrier comprises a single layer of web material folded along a first and second fold line to form a folded section comprising at least 4 layers and a non-folded section comprising at least 2 layers.

## Description

### TECHNICAL FIELD

The present invention relates to absorbent articles comprising cuffs. Such articles are for example baby or adult diapers, pants or sanitary napkins. Typically said absorbent articles comprise single-layer cuffs being folded at its top to include one or more elastic elements. The present invention relates to absorbent articles comprising double-layer cuffs, which are configured to reduce risk of side leakage and to improve comfort.

### BACKGROUND

Absorbent articles for personal hygiene are designed to absorb and contain bodily exudates, such as a large quantity of urine. Non-limiting examples of disposable absorbent articles include diapers, pants, training pants, pADL, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet, an absorbent core, and elasticized cuffs among other layers.

Side leakage is a common problem for absorbent articles. Gushes of urine discharge can temporarily flood the surface of the absorbent article, leaking over the side margin. To adress this problem, others have incorporated elastic gathering elements in various ways along the lateral edge of the absorbent article.

Some articles have gathered elastic members along the side margins. The elastic members are placed sufficiently away from the relatively stiff absorbent core to allow contraction during wear. The gathered edges can create edge barriers to resist urine leakage at the sides. However, with these designs, the side elastic members are in physically close proximity to the elastic of the underwear of the user. The elastics of the underwear frequently dominate the elastics of the absorbent article, rendering them ineffective at preventing side leakage.

Conventional barrier cuffs typically include a single cuff of which examples are disclosed in Japanese Patent Laid-Open publications 235501/1988, 280951/1991 and 184622/1993.

Such a single cuff has a proximal edge attached to the absorbent article and an elasticized distal edge opposite to the proximal edge, attempting to provide a seal against the body of a wearer to prevent the flow of body exudates. However, a single barrier cuff often fails to provide a sufficient seal between the distal edge of the cuff and the wearer's body. The lack of a sufficient seal results in leakage of exudates, especially runny fecal matter. Moreover, the single barrier cuff is not particularly effective enough to resist forceful and repetitive excretion of bodily exudates. The leakage of exudates past the barrier cuff results in soiling the clothing, bedding, and person.

In an attempt to overcome the above identified disadvantages of single barrier cuffs, a double layer barrier cuff structure has been disclosed in e.g. US Patent Laid-Open publications No. 8,795,250 B2 (First Quality), U.S. Pat. No. 10,543,131 (P&G) and U.S. Pat. No. 9,301,889 (P&G).

These patent publications disclose an absorbent article comprising a pair of laterally opposite outer cuffs and a pair of laterally opposite inner cuffs. The combination of one outer cuff with one inner cuff at each of sides of the article functions as a double barrier cuff. Because such a double cuff structure provides a double seal to prevent the flow of bodily exudates, the ability to prevent leakage of discharged excreta is enhanced in comparison with singe barrier cuffs. However, such a double barrier cuff still suffers from the drawback that the distal edge of each cuff hardly follows the movement of the wearer's body in order to continue to contact with the wearer's body during use of the article. The separation of the distal edge of the barrier cuff from the wearer's body can result in leakage of discharged excreta.

In conventional designs, as disclosed above, bunching of absorbent material can cause material of the side barriers to be pushed into the fluid path, preventing the absorbent core from working effectively. Furthermore, many of these designs are uncomfortable to wear because the elastics create a harsh edge which rubs against the wearer.

There remains a continued need for an absorbent article having improved side leakage protection and increased wearing comfort, minimizing the impact of the elastic elements in leg cuffs on the wearer's skin.

### SUMMARY

In a first aspect, the invention relates to a disposable absorbent chassis having a longitudinal direction along a longitudinal central axis, a transverse direction along a transverse central axis, a user-facing side and a garment-facing side, a front waist portion, a rear waist portion and a crotch portion disposed longitudinally between the front waist portion and the rear waist portion. The disposable absorbent chassis comprises a first longitudinal side edge and a second longitudinal side edge and comprises a liquid-pervious topsheet, a backsheet, a liquid absorbent core disposed between the liquid-pervious topsheet and the backsheet. The backsheet comprises a liquid-impervious film layer and optionally an outer cover layer wherein the liquid-impervious film layer has a first longitudinal edge, a second longitudinal edge and a liquid-impervious film layer width measured in the transverse direction. The outer cover layer has a first longitudinal edge and a second longitudinal edge and an outer cover width measured in the transverse direction. The disposable absorbent chassis further comprises a pair of opposed elasticized outer leg barriers disposed on either side of the longitudinal central axis comprising at least one elastic member. Furthermore the disposable absorbent chassis comprises a pair of longitudinal standing gathers disposed on either side of the longitudinal central axis, wherein each longitudinal standing gather has a proximal end attached to the chassis, a distal end extending towards the longitudinal central axis and comprises a single layer of web material wherein each standing leg gather has a lateral flap extending transversely outward toward one of the longitudinal side edges of the chassis. The disposable absorbent chassis may have a waist barrier disposed on either side of the transverse central axis, wherein each waist barrier has a proximal end attached to the chassis, a distal end extending towards the transverse central axis and comprises a single layer of web material. The single layer of web material of each of the longitudinal standing gathers and/or waist barriers is folded along a first fold line to form a standing gather and/or waist barrier with at least 2 layers comprising an inner layer and an outer layer, wherein each standing gather and/or waist barrier is folded along a second fold line to form a folded section comprising at least 4 layers and a non-folded section comprising at least 2 layers.

In a second aspect, the disclosure relates to a disposable absorbent article, preferably an absorbent diaper or an absorbent pant, comprising a disposable absorbent chassis, in which the elastic members of the elasticized outer leg barriers extend between the back ear panels and the front ears, or between the front elasticized belt and the back elasticized belt.

In a third aspect, the disclosure relates to a re-usable outer cover and a disposable absorbent chassis.

### BRIEF DESCRIPTION OF THE FIGURES

**FIG. 1** illustrates a plan view of an exemplary disposable absorbent chassis in an extended state according to an embodiment of the present invention
**FIG. 2A to 2G** are transverse sectional views of the chassis of FIG. 1, as viewed along A-A, and represent different embodiments of the first and second longitudinal standing gathers, waist barriers and elasticized outer leg barriers.
**FIG. 3** is a top plan view of a disposable diaper in a flat configuration, including the chassis of the present embodiments.
**FIG. 4** is a top plan view of a disposable pants in a flat configuration, including the chassis of the present embodiments.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

The term "absorbent article" refers to a device that absorbs and contains liquid, and more specifically, refers to a device that is placed against or in proximity to the body of the wearer to absorb and contain the various wastes/exudates discharged from the body. Absorbent articles include but are not limited to diapers, adult incontinence briefs, training pants and diaper holders and liners.

The "absorbent core" is the absorbent structure disposed between the topsheet and the backsheet of the chassis and is capable of absorbing and retaining liquid body exudates. The size and the absorbent capacity of the absorbent core should be compatible with the size of the intended wearer and the liquid loading imparted by the intended use of the chassis. Further, the size and the absorbent capacity of the absorbent core can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent core. It can contain embossed channels, channels substantially free of material, channels with lower basis weight than the rest of the core, etc.

The terms "activated" and "pre-activated" refer to a process of mechanically deforming a material in order to increase the extensibility of at least a portion of the material. A material may be activated or pre-activated by, for example, incrementally stretching the material in at least one direction.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help to prevent the liquid from pooling and collecting on the portion of the absorbent garment positioned against the wearer's skin, thereby reducing the feeling of wetness by the wearer. Preferably, the surge management portion is positioned between the top sheet and the retention portion.
The term "adhesive" as used herein is intended to refer to any suitable hot melt, water or solvent borne adhesive that can be applied to a surface in the required pattern or network of adhesive areas. Accordingly, suitable adhesives include conventional hot melt adhesives, pressure-sensitive adhesives and reactive adhesives (i.e., polyurethane).

The terms "adhesively bonded" or "adhesively laminated" refer to a laminate wherein an adhesive is used to bond an elastomeric material to at least one cover layer.

The term "attached" refers to elements being connected or united by fastening, adhering, bonding, or by any other method suitable for connecting the elements together and to their constituent materials. Many suitable methods for attaching elements together are well-known, including adhesive bonding, pressure bonding, thermal bonding, ultrasonic bonding, mechanical fastening, etc. Such attachment methods may be used to attach elements together over a particular area either continuously or intermittently.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

The terms "back waist portion", "back section" or "back portion" are used herein as synonyms and refer to the area of the absorbent article which is contact with the back of the wearer when the absorbent article is worn.

As used herein, the " skin-facing" or "bodyside" or " body-facing" or "user facing" side or surface means that surface of the article or component which is intended to be disposed toward or placed adjacent to the body of the wearer during ordinary use, while the "outward", "outward-facing", "garment-facing" or "garment" side or surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

"Breathable" as used herein is the ability of the structure referred to (e.g. a layer(s), laminate etc.) of allowing water vapor to permeate therethrough and typically that the structure referred to has a water vapor transmission rate (WVTR) of at least 1500 grams/m² - 24 hours, preferably at least 2500 grams/m² - 24 hours, according to the method described herein.

A "chassis" of a disposable absorbent articles can include a liquid pervious topsheet, a backsheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The chassis comprises a front waist portion, a back waist portion, an intermediate crotch portion which interconnects the front and back waist portions. When used herein, reference to a "front" portion refers to that part of the chassis which is generally located on the front of a wearer when in use. Reference to the "back" portion refers to the portion of the chassis generally located at the back of the wearer when in use, and reference to the "crotch" portion refers to that portion which is generally located between the legs of a wearer when in use. The crotch portion is an area where repeated fluid surge typically occurs.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "diaper" refers to an absorbent article generally worn by infants or incontinent persons about the lower torso and having the general form of a sheet, different portions of which are fastened together to encircle the waist and the legs of the wearer.

The term "disposable" is used herein to describe absorbent articles that generally are not intended to be laundered or otherwise restored or reused as an absorbent article i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "extensible" refers to the property of a material, wherein: when a biasing force is applied to the material, the material can be extended to an elongated length of at least 110% of its original relaxed length (i.e., can extend 10%), without a rupture or breakage that renders the material unusable for its intended purpose. A material that does not meet this definition is considered inextensible. In some embodiments, an extensible material may be able to be extended to an elongated length of 125% or more of its original relaxed length without rupture or breakage that renders the material unusable for its intended purpose. An extensible material may or may not exhibit recovery after application of a biasing force. Throughout the present disclosure, an extensible material is considered to be "elastically extensible" if, when a biasing force is applied to the material, the material can be extended to an elongated length of at least 110% of its original relaxed length (i.e., can extend 10%), without rupture or breakage which renders the material unusable for its intended purpose, and when the force is removed from the material, the material recovers at least 40% of its elongation. In various examples, when the force is removed from an elastically extensible material, the material may recover at least 60%, or at least 80%, of its elongation.

The term "film" refers to a sheet-like material wherein the length and width of the material far exceed the thickness of the material. Typically, films have a thickness of about 0.5 mm or less.

The term "flat configuration" or "laid-flat state" or "fully stretched state" or "extended state" or flat-out configuration is intended to refer to the article when it is flattened into a plane or is substantially flattened into a plane and is used in contrast to when the article otherwise positioned, such as when the article is folded or shaped in or for use by a wearer.

As used herein, the term "impermeable" or "impervious" generally refers to articles, chassis and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The term "lateral" or "transverse" refers to a direction running at a 90 degree angle to the longitudinal direction and when combined with the term "substantially" includes directions within ±45° of the lateral direction.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The term "longitudinal" refers to a direction running parallel to the maximum linear dimension of the article and when combined with the term "substantially" includes directions within ±45° of the longitudinal direction.

The term "nonelastic" or "inelastic" refers to any material which does not fall within the definition of "elastic" above.

The term "nonwoven substrate/fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "recovery" refers to ability of a material to return to its original size after it has been stretched.

The term "refastenable" refers to the property of two elements being capable of releasable attachment, separation, and subsequent releasable reattachment without substantial permanent deformation or rupture.

The terms "releasably attached," "releasably engaged," and variations thereof refer to two elements being connected or connectable such that the elements tend to remain connected absent a separation force applied to one or both of the elements, and the elements being capable of separation without substantial permanent deformation or rupture. The required separation force is typically beyond that encountered while wearing the absorbent garment. The term "re-usable outer cover" or "re-usable shell", refers to a component of a disposable absorbent article that is adapted to be worn about the lower torso of a user, and is configured to support and hold a disposable absorbent insert, such as the absorbent chassis described herein. The reusable cover permits removal and replacement of the absorbent insert without substantial destruction of the components of the outer cover that are necessary to the functionality of the outer cover. The reusable outer cover may comprise materials and have a construction that enable it to be re-used and/or may be washed in order to be used more than once.

Further, a chassis can comprise "standing gathers" or "containment flaps" or "barrier cuffs" or "leg cuffs". The standing gathers are generally thought to be particularly well suited for the containment of fecal matter and to prevent the lateral flow of liquid waste until such time as the liquid waste can be absorbed by the absorbent article. Many constructions of standing gather are known. Such standing gather generally comprises a proximal portion intended to be attached to the chassis, and an opposed distal portion which is generally not attached to the chassis along at least a portion of its length. An elastic member is generally located on or adjacent a distal edge to provide elasticity to the standing gather, to assist in maintaining the containment flap in an upright condition and in maintaining a sealing relationship between the distal edge of the standing gather and the body of a wearer during use. The elastic member is generally located between two layers of material so that the elastic does not come into contact with the body of a wearer. The standing gather may be manufactured from any of a wide variety of materials such as woven, non-woven, spunbonded, carded, cast, blown or the like, or combinations or laminations thereof. The materials may include any of a variety of compositions including but not limited to polypropylene, polyester, rayon, nylon, foams, plastic films, formed films, and elastic foams. To improve containment, the material may be hydrophobic, or at least partially liquid impervious. A number of manufacturing techniques may be used to manufacture the containment flaps.

By "substantially", it is meant at least the majority of the structure referred to.

Use of the term "substrate" includes, but is not limited to, woven or nonwoven webs, porous films, ink permeable films, paper, composite structures, or the like.

"Superabsorbent polymer particles" or "SAPs" refer to water-swellable, water-insoluble organic or inorganic materials capable, under the most favorable conditions, of absorbing at least about ((10)) times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between ((10))0 to 800 µm, preferably between 300 to 600 µm, more preferably between 400 to 500 µm.

Superabsorbent materials (e.g., superabsorbent polymers) suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g., saline with 0.9 wt. % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system and may optionally be employed in other components or portions of the absorbent article. The superabsorbent material may be included in the absorbent layer or other fluid storage layer of the absorbent article of the present invention in an amount up to ((10))0% by weight. Typically, the superabsorbent material, when present, will be included in an amount of from about 30% to about 70% by weight, based on the total weight of the absorbent layer.

The term "trilateral" refers to a shape having three sides and three corners. In certain embodiments, the trilateral shape refers to a triangular shape. In certain embodiments, the corners can be rounded corners. In addition, in certain embodiments, the sides of the trilateral can be flexible.

Embodiments of the articles and processes according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ABSORBENT CHASSIS

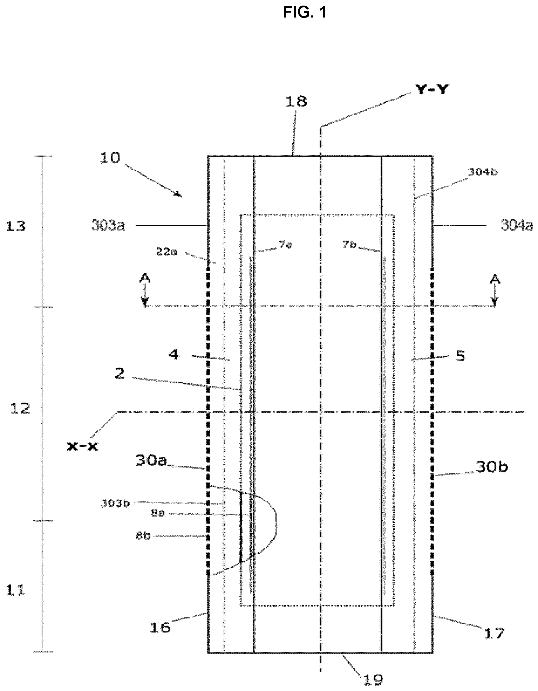

FIG. 1 illustrates a plan view of an exemplary disposable absorbent chassis (10) in an extended state according to an embodiment of the present invention. As can be seen, the chassis (10) comprises a longitudinal central axis Y-Y and a transverse central axis X-X. The longitudinal central axis Y-Y extends along the length of the chassis (10) and the transverse central axis X-X extends along the width of the disposable chassis (10).

The chassis (10) comprises a liquid-pervious topsheet (1) defining at least a portion of the user-facing or skin-facing side of the chassis (10), a backsheet (3) defining at least a portion of the garment-facing side of the chassis (10) and a liquid absorbent core (2) disposed between the liquid-pervious topsheet (1) and the backsheet (3). While the chassis (10) is illustrated in FIG. 1 as having a generally rectangular shape, the chassis (10) could have other shapes or contours, as desired. The chassis (10) comprises a front waist portion (13), a back waist portion (11) and an intermediate crotch portion (12) which interconnects the front and back waist portions. The chassis (10) has a perimeter that is defined, at least in part, by a first longitudinal side edge (16), a second longitudinal side edge (17), a first transverse edge (18), and a second transverse edge (19).

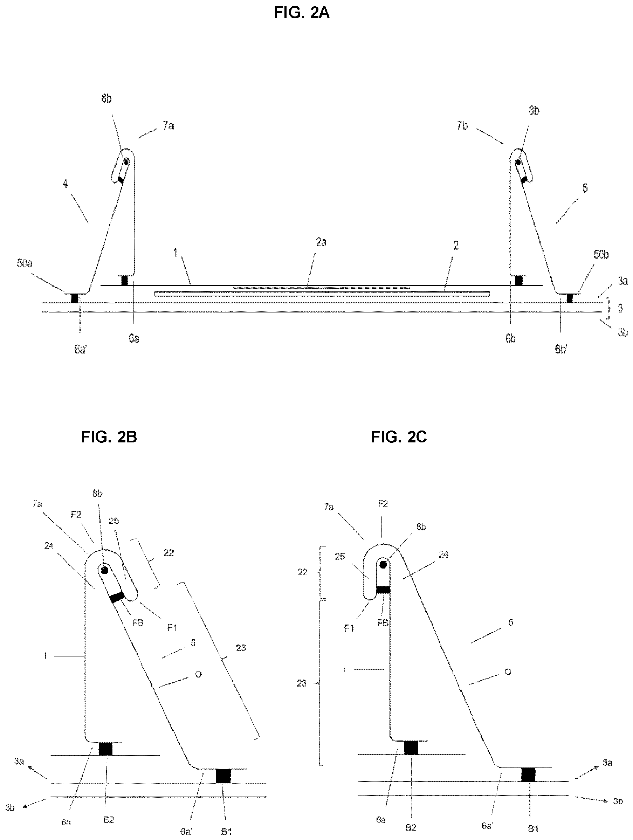

The backsheet (3) is water impervious or hydrophobic and can include a liquid impervious film layer (3a), a laminate of a liquid impervious film layer (3a) and an outer cover layer (3b), as shown in FIG. 2A, or any hydrophobic layer as is known in the art. In the embodiments in which a laminate of a liquid impervious film layer (3a) and an outer cover layer (3b) is used as backsheet (3), the liquid impervious film layer (3a) comprises a first surface usually facing to the wearer's skin, a second surface, usually facing to the wearer's garment, a first longitudinal edge (303a), a second longitudinal edge (304a), a first transverse edge (305a) and a second transverse edge (306a) and the outer cover layer (3b) of the backsheet (3) comprises a first surface, usually facing to the wearer's skin, a second surface, usually facing to the wearer's garment, a first longitudinal edge (303b), a second longitudinal edge (304b), a first transverse edge (305b) and a second transverse edge (306b).

The absorbent core (2) preferably comprises an absorbent material, said absorbent material comprising cellulose fibers and/or superabsorbent polymers, preferably wherein said absorbent material is contained within at least one core wrap substrate enclosing said absorbent material therein, typically wherein the superabsorbent polymers are in the form of particles or granules, fibers, and mixtures thereof. The core wrap is typically a nonwoven selected from the group consisting of spunbond (S), spunbond-meltblown (SM), spunbond-metlblown-spunbond (SMS), spunbond-meltblown-metlblown-spunbond (SMMS), spunbond-spunbond-metlblown-spunbond (SSMS), spunbond-spunbond-metlblown-spunbond-spunbond (SSMSS), and combinations thereof. The core wrap may also, or alternatively, comprise a carded nonwoven preferably a carded thermobonded nonwoven wherein the thermobonds are formed by calendering (i.e. a carded nonwoven free of air-through bonding).

The absorbent article may further comprise an acquisition distribution layer (ADL) (2a) positioned between the topsheet (1) and an upper layer of the core wrap. The ADL may be in good and/or direct contact with both the topsheet (1) and an upper layer of the core wrap over the majority of its surface area. ADLs customary in the art may be used such as nonwovens selected from spunbond or carded thermobonded whether air-through bonded or calendered. Also ADLs comprising cellulosic fibers and/or polylactide are suitable.

The absorbent core may further comprise one or more channels substantially free of absorbent material, typically meaning less than 15%wt or less than 5% of absorbent material within said channels. Preferably, a top layer of the core wrap is bonded to a bottom layer of the core wrap in one or more attachment zones corresponding to said channels thereby allowing the channels to be substantially free of absorbent material or even essentially entirely free of absorbent material. The bonding may be achieved via adhesive and/or mechanical bonding.

The disposable absorbent chassis (10), as exemplified in FIG. 2A, may comprise first and second longitudinal standing gathers (4, 5) disposed on either side of the longitudinal central axis Y-Y. Each of the first and second elasticized longitudinal standing gather (4,5) comprises a first proximal portion (6a) joined to the topsheet (1) and/or a second proximal portion (6a') joined to the backsheet (3), a distal portion (7a,7b) standing away from the topsheet (1) and elasticized by means of at least one elastic member (8b) enclosed within a substrate. Outboard of its attachment to the topsheet (1), each standing leg gather (4,5) has a lateral flap (50a, 50b) that extends transversely outward toward the longitudinal edges (16, 17) of the chassis (10), respectively. The at least one elastic member (8b) may be attached to the substrate in an stretched condition, joined to the substrate while the substrate is in a gathered state, or joined to the substrate and then elasticized or shrunk, for example with the application of heat, so that elastic retractive forces are imparted to the substrate, or a combination of these methods. The resulting elastic retractive forces will cause the substrate forming the standing gather to gather at the distal portion (7a, 7b) and cause the standing gather to extend away from the skin-facing surface of the topsheet (1) (e.g., see FIG. 2A) such that the standing gather (4, 5) is at least partially extensible in the longitudinal direction.

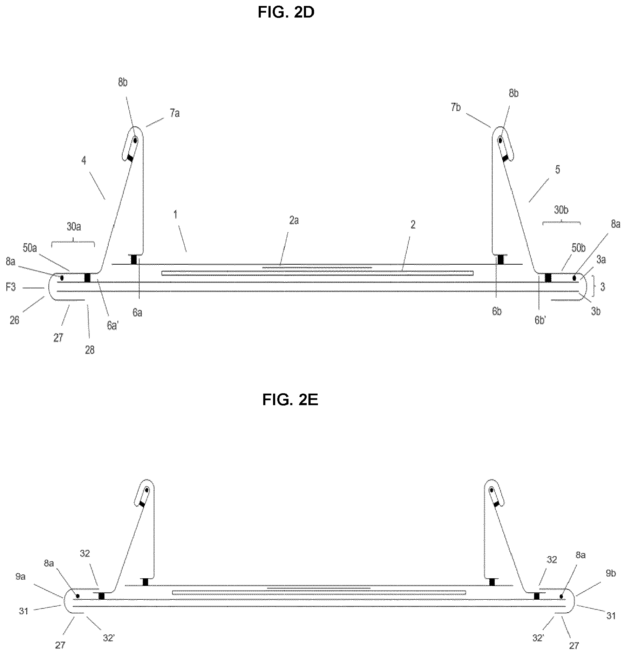

The disposable absorbent chassis (10), as exemplified in FIG. 2D, includes a pair of opposed elasticized outer leg barriers (30a, 30b) disposed on either side of the longitudinal central axis Y-Y. Each of the outer leg barriers (30a, 30b) has a proximal edge (303b, 304b) proximal to the longitudinal sides of the core, and a distal edge (26, 31) disposed generally at or adjacent the first longitudinal side edge (16), or the second longitudinal side edge (17) of the chassis, respectively. The outer leg barriers (30a, 30b) may extend the entire longitudinal dimension of the longitudinal side edges (16, 17), or only a portion thereof. The elastic members (8a) of the outer leg barriers (30a, 30b) extend at least along the crotch portion and may also extend at least part of the front and/or back waist portions of the chassis (10). The outer leg barriers (30a, 30b) will be described in more detail with reference to the various embodiments shown in FIG. 2D-2G.

Absorbent articles herein may further comprise additional components such as frontal (typically inelastic) panels; elastically elongatable panels joined to the chassis; one or more skin care lotions on the topsheet; one or more odor control compositions and/or fragrances contained in one or more layers beneath the topsheet; and other components common in the art of diapers and pants.

### TOPSHEET

The topsheet (1) refers to a liquid permeable material sheet forming the inner cover of the chassis of an absorbent article and which in use is placed in direct contact with the skin of the wearer. The topsheet is typically employed to help isolate the wearer's skin from liquids held in the absorbent structure. The materials suited as top sheet materials should be soft and non-irritating to the skin and be readily penetrated by body fluid, e.g., urine or menstrual fluid.

The topsheet (1) can comprise a nonwoven material, e.g., spunbond, meltblown, carded, hydroentangled, wetlaid or any other type of nonwoven. Suitable nonwoven materials can be composed of man-made fibres, such as polyester, polyethylene, polypropylene, viscose, rayon etc. or natural fibers, such as wood pulp or cotton fibres, hemp, and the like, or other natural or naturally-derived fibers, or from a mixture of two or more natural or man-made fibres. The top sheet material may further be composed of two fibres, which may be bonded to each other in a bonding pattern.

Further examples of topsheet materials are woven materials, apertured formed thermoplastic films, apertured plastic films, hydroformed thermoplastic films, porous foams, reticulated foams, reticulated thermoplastic films, and thermoplastic scrims.

Further, the topsheet (1) may be composed of a substantially hydrophobic material, and the hydrophobic material may optionally be treated with a surfactant or otherwise processed to impart a desired level of wettability and hydrophilicity. Such surfactants may be applied to the entire topsheet or may be selectively applied to particular sections of the topsheet, such as the medial section along the longitudinal centerline of the absorbent article, to provide greater wettability of such sections. The topsheet may further include a composition applied thereto that is configured to be transferred to the wearer's skin for improving the skin health of the wearer.

### BACKSHEET

The backsheet (3) refers to a material forming a liquid impervious cover of the chassis of the absorbent article. The backsheet prevents the exudates contained in the absorbent core from wetting articles such as bedsheets and overgarments which contact the disposable absorbent article. The backsheet may be a composite layer composed of multiple components assembled side-by-side or laminated. The backsheet may be the same or different in different parts of the chassis. At least in the area of the absorbent core, the backsheet comprises a liquid impervious material in the form of a thin plastic film, e.g., a polyethylene or polypropylene film, a nonwoven material coated with a liquid impervious material, a hydrophobic nonwoven material, which resists liquid penetration, or a laminate of a plastic film and a nonwoven material. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials, in such laminates, the polymeric film can have the same or a different width than the nonwoven material.

### LONGITUDINAL STANDING GATHERS

As exemplified in Fig. 2A-2C, the longitudinal standing barriers (4, 5) preferably comprise a single layer of web material which is folded along a first fold (F1) line to form a standing gather with at least 2 layers comprising an inner layer (20) and an outer layer (21).

In a preferred embodiment, as exemplified by FIG. 2A-2C, each of the longitudinal standing gathers (4, 5) is folded along a second fold line (F2) to form a folded section (22) comprising at least 4 layers and a non-folded section (23) comprising at least 2 layers.

In an embodiment, as exemplified in Fig. 2B, the folded section (22) comprises a first section (24) comprising at least 2 layers and a second section (25) comprising at least 2 layers in which the garment-facing side of the first section (24) is bonded to the user-facing side of the second section (25) at a folded section bond site (FB). Preferably, at least one elastic element (8b) is bonded to the garment-facing side of the first section (24) and/or to the user-facing side of the second section (25) between the folded section bond site (FB) and the second fold line (F2). Even more preferably, each longitudinal standing gather (4, 5) comprises at least 2 elastic elements. The elastic elements may be flat elastics.

In an embodiment, as exemplified in Fig. 2C, the folded section (22) comprises a first section (24) comprising at least 2 layers and a second section (25) comprising at least 2 layers in which the user-facing side of the first section (24) is bonded to the garment-facing side of the second section (25) at a folded section bond site (FB). Preferably, at least one elastic element (8b) is bonded to the user-facing side of the first section (24) and/or to the garment-facing side of the second section (25) between the folded section bond site (FB) and the section fold line (F2). Even more preferably, each longitudinal standing gather (4, 5) comprises at least 2 elastic elements. The elastic elements may be flat elastics.

In an embodiment, as exemplified in Fig. 2B-2C, the user-facing side of the outer layer (O) of the non-folded section (23) is attached to the user-facing side of the liquid impervious film layer (3a) at a first bond site (B1) and wherein the user-facing side of the inner layer (I) of the non-folded section (23) is bonded to the user-facing side of the liquid-pervious topsheet (1) at a second bond site (B2).

In a preferred embodiment, as exemplified in Fig. 2B-2C, each longitudinal standing gather (4, 5) has an inner layer (I), an outer layer (O), a distal end (7a, 7b), a first proximal position (6a, 6b), a second proximal position (6a', 6b'), a first bond site (B1), a second bond site (B2), a lifted position and a flat position, and a cross-sectional area that is a generally trilateral shape in the lifted position. The cross-sectional area is defined by the distal end (7a, 7b), the inner layer (I), the outer layer (O), a first proximal position (6a, 6b), a second proximal position (6a', 6b'), the first bond site (B1) and the second bond site (B2). Advantageously, this trilateral shaped, cross-sectional area provides for better guidance of exudates towards the point at which the inner layer and outer layer of the longitudinal standing gathers have the highest separation when compared to quadrilateral shaped, cross sectional areas or conventional longitudinal standing gathers without separation between inner and outer layer. This point of highest separation provides for the best barrier function as the exudates needs to go through 2 separated layers in between which an air gap is present. This air gap in between the inner layer and the outer layer of the longitudinal standing gathers could serve as temporary storage for exudates penetrated through the inner layer and could allow this liquid to penetrate the topsheet and flow back to the absorbent core material.

### ELASTICIZED OUTER LEG BARRIERS

In an embodiment, as exemplified in FIG. 2D, each of the elasticized outer leg barriers (30a, 30b) comprises a substrate folded about a third fold line (F3) towards the longitudinal central axis such that a folded portion (27) extends between a distal folded edge (26) and a substrate edge (28), in which the substrate edge (28) is located transversely inward from the distal folded edge (26), and wherein the folded portion (27) comprises a user-facing side and a garment-facing side, preferably wherein the substrate of the elasticized outer leg barriers (30a, 30b) comprises the single layer of web material folded towards the garment-facing side of the absorbent chassis (10). Advantageously, such configuration provides for an extra safety barrier against side leakage in case exudates would migrate underneath the longitudinal standing gathers towards the side edges of the absorbent chassis.

In another embodiment, as exemplified in FIG. 2E, the outer leg barriers (30a, 30b), comprise a separate wrapper element (9a, 9b) that is a folded substrate joined to the one or both layers of the backsheet (3). The wrapper element (9a, 9b) has a folded distal edge (31), a first proximal edge (32) and a second proximal edge (32'). The folded portion (27) of the outer leg barriers (30a, 30b) spans the distance between the folded distal edge (31) and the second proximal substrate edge (32') of the wrapper element (9a, 9b). The elastic members (8a) are disposed within the folded portion, at or adjacent the distal folded edge (31). The wrapper element may be joined to the outer cover layer (3b), and optionally the liquid impervious film layer (3a) in various different configurations explained later. The outer leg barriers (30a, 30b), including the respective wrapper elements (9a, 9b), are bonded to the lateral flaps (50a, 50b) of the elasticized longitudinal standing gathers (4, 5). Advantageously, such configuration provides for an extra safety barrier against side leakage in case exudates would migrate underneath the longitudinal standing gathers towards the side edges of the absorbent chassis.

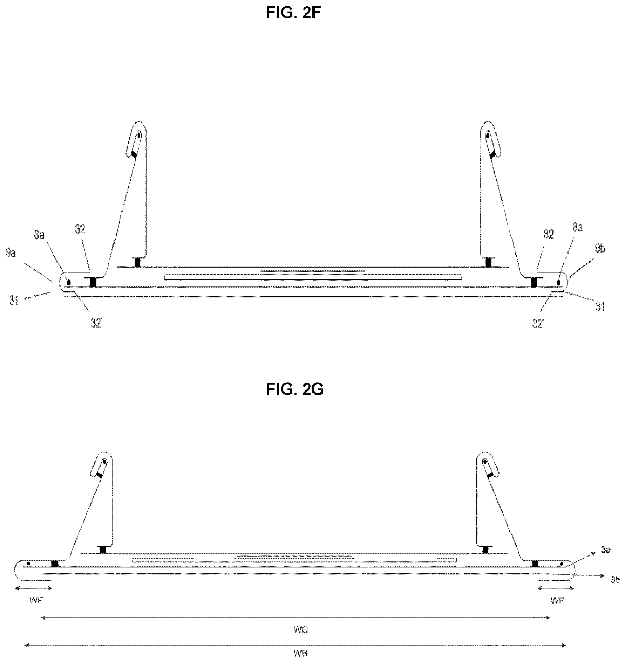

The wrapper elements (9a,9b) may comprise a substrate that is the same as or is different from the outer cover layer 3b, the standing gather (9a,9b), or both, such as a nonwoven or a nonwoven-film laminate. The wrapper element (9a,9b) may be joined to the outer cover (3b), the liquid impervious film layer (3a), or both, in any of various configurations. For example, the wrapper element (9a,9b) may be joined to a garment-facing side of the outer cover (3b) as shown in FIG. 2E, to the user-facing surface of the outer cover 3b as shown in FIG. 2F.

The wrapper element (9a, 9b) may be joined to the outer cover (3b), the liquid impervious film layer (3a), or both by any suitable means, such as adhesive bonding, mechanical bonding, or a combination thereof. In all described configurations the outer leg barriers (30a, 30b) may be joined to the lateral flaps (50a,50b) of the elasticized longitudinal standing gathers (4, 5).

If the backsheet is formed of only one layer of liquid impervious or hydrophobic material, the wrapper element (9a, 9b) is joined as described to this liquid impervious or hydrophobic layer (3a): on the user-facing side, on the garment-facing side or sandwiching the layer.

In an embodiment, the user-facing side of the folded portion (27) is attached to the garment-facing surface of the outer cover layer (3b); and/or wherein the user-facing side of the folded portion (27) is attached to the garment-facing surface of the liquid-impervious film layer (3a); and/or wherein the garment-facing side of the folded portion (27) is attached to the user-facing side of the outer cover layer (3b).

In a preferred embodiment, as exemplified in FIG. 2G, the folded section (27) of the elasticized outer leg barriers (30a, 30b) has a folded section width (WF) measured in the transverse direction in which the outer cover width (WC) is less than the liquid-impervious film layer width (WB) and greater than the liquid-impervious film layer width (WB) minus 2 times the folded section width (WF). This results in the following formula: (WB - (2 x WF)) ≤ WC < WB. Advantageously, this would allow for material cost savings without loss of performance and/or comfort. The outer cover width (WC) is the distance between the longitudinal side edges of the outer cover (3b) measured parallel to the lateral central axis (X-X), the liquid-impervious film layer width (WB) is the distance between the longitudinal side edges of the liquid-impervious film layer (3a) measured parallel to the lateral central axis (X-X) and the folded section width (WF) is the distance between the distal folded edge (26, 31) and the second proximal substrate edge (28, 32').

In an embodiment, at least one elastic member (8a) is disposed within the folded portion (27) at the distal folded edge (26, 31) of the elasticized outer leg barrier (30a, 30b). Preferably, at least 2 elastic members are disposed within the folded portion (27) at the distal folded edge (26, 31) of the elasticized outer leg barrier (30a 30b).

In an embodiment, the substrate of the elasticized outer leg barriers (30a, 30b) has one or both of: a TS7 value between 2 dB V² rms and 8 dB V² rms measured according with the Emtec test method; and a TS750 value between 3 dB V² rms and 20 dB V² rms measured according with the Emtec test method; and/or wherein the lateral flaps () of the elasticized longitudinal standing gathers (), has one or both of: a TS7 value between 2 dB V² rms and 8 dB V² rms measured according with the Emtec Tissue Softness test method; and a TS750 value between 3 dB V² rms and 20 dB V² rms measured according with the Emtec Tissue Softness test method.

### THE ABSORBENT ARTICLE

Another aspect of this invention is an absorbent article, such as an absorbent diaper (100) and/or absorbent pant (1000), that includes a chassis (10) as described herein. The chassis (10) could be a separate sub-assembly that is joined with one or more additional components of the absorbent article, it can be formed integrally with one or more components of the absorbent articles, or a combination thereof. It will be understood that these absorbent articles can optionally contain other features and elements such as a fastening system, a pair of front ears, a pair of back ears, a front belt, a back belt, elastic waist features, acquisition/distribution layers, front and back barriers , a pair of elastic or inelastic panels, front and back belts, or any other elements known in the art.

The absorbent article typically comprises a front region (F), a back region (B), and a crotch region positioned between the front region (F) and back region (B) such that the longitudinal axis (y) crosses each of said front region (F), crotch region and back region (B) generally in this order. The crotch region of the article typically being positioned to fit between the wearer's legs when the article is worn by a subject.

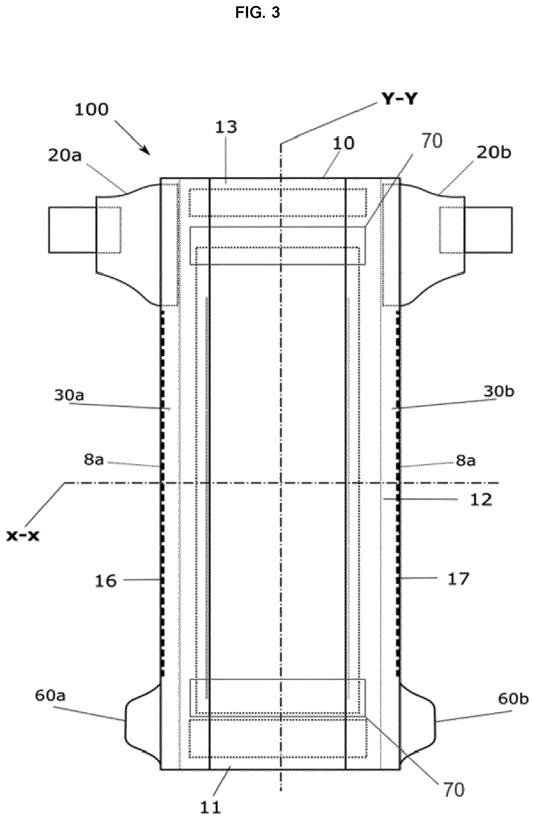

Referring to FIG. 3, in one aspect, a disposable absorbent diaper (100), such as a diaper intended for use by an adult, a baby, or a child, includes the chassis (10) of the present invention. In this aspect, the chassis (10) is formed integrally with the diaper (100), so that the outer cover of the chassis (10) forms the outer cover of the diaper (100). The chassis may further include an acquisition/distribution layer (2a) between the core (2) and the topsheet (1) and/or between the core (2) and the backsheet (3). The diaper (100) may include a pair of opposed back ear panels (20a, 20b) joined to the longitudinal side edges (16, 17) of the chassis (10) at the back waist portion (13), and extending outward. The disposable absorbent article (100) may include a pair of opposed frontal ears (60a, 60b) which are joined to longitudinal side edges (16,17) the chassis (10) in the front waist portion (11) and extend outward therefrom. The front ear panels (60a, 60b), back ear panels (20a, 20b) or both may be elastic, inelastic, or a combination thereof. The ear panels may include one or more elastic or inelastic materials such as a nonwoven, a film, a foam, a filament or strand, or a combination thereof, such as a laminate of two or more materials. For example, the ear panels may include a laminate of an elastic film and a nonwoven material that is extensible in the transverse direction. The ear panels may be attached to the chassis by adhesive, mechanical, thermal bonding, or a combination thereof. One or more fastening members may be joined, directly or indirectly, to the front ear panels, back opposed ear panels, or both. For example, mechanical fasteners may be provided at or near the distal edges of the back ear panels (20a, 20b), that are configured to releasably engage with a corresponding material on front waist portion (11), to enable the diaper (100) to be fastened about the waist of a user. Exemplary fastening means include tape tab fasteners, snaps, pins, belts, hooks, buckles, "hook/mushroom"- and-loop fasteners (e.g. VELCRO^{®}-type fasteners) and the like. The diaper (100) may also include other features such as elastic waist features or barriers in the back portion (13), front portion (11), or both.

The outer leg barriers (30a, 30b) of the chassis (10) extend longitudinally on the diaper (100). The elastic members (8a) of the elasticized outer leg barriers (30a, 30b), extend over the crotch portion of the chassis (10) and at least into part of the front waist portion (11) and back waist portion (13). For example, referring to FIG. 3, the elastic members (8a) are shown extending between the back ear panels (20a, 20b) and optional frontal ears (60a, 60b), passing through the crotch portion (12). When the diaper is fastened about the waist of the user, the elasticized outer leg barriers (30a, 30b) can form a gasket about the legs of the user to control or prevent liquid leakage.

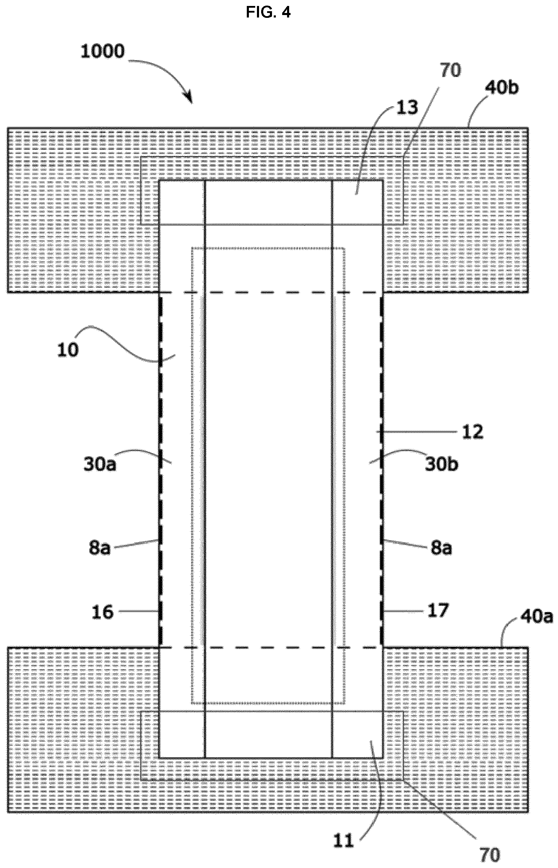

In another aspect, a disposable absorbent pant (1000), such as an adult pant, a child pant, or a baby pant, may include a chassis (10) as described herein. For example, referring to FIG. 4, disposable pants (1000) may comprise a front elasticized belt (40a) adjacent a front waist portion (11) and a back elasticized belt (40b) adjacent a back waist portion (13) of the chassis (10), the elasticized belts (40a, 40b) are extensible in the transverse direction (e.g., parallel to transverse axis X-X).. The front and back elasticized belts (40a, 40b) may comprise an inner nonwoven layer, an outer nonwoven layer and one or more elastic materials (i.e., elastic film, elastic strand) disposed between the inner and outer nonwoven layers. The inner and outer nonwoven layers may be joined to each other and/or to the elastics using adhesive, thermal, or mechanical bonds, or a combination thereof. The front elasticized belt (40a) and a back elasticized belt (40b) can be sealed together at lateral side seams to form an underwear-resembling product that can be pulled up over the legs of a user and may be removed either by pulling it down in the opposite direction or by tearing the side seams. It will be understood that a fastener system can be provided as an alternative to the side seam, so that the plant is refastenable.

The chassis (10) may be joined directly or indirectly with the front and back belts (40a, 40b) and extending longitudinally therebetween to form an absorbent article. For example, the disposable absorbent pant (1000) may have an outer cover that extends the full length of the pant (1000), and each of the front belt (40a), the back belt (40b), and the chassis (10) are joined to the outer cover. As an alternative, the chassis (10) may be directly joined to the front belt (40a) and the back belt (40b), extending therebetween, with the outer cover (3b) of the chassis (10) forming a portion of the outer cover of the pant. The elastic members (8a) of the elasticized outer leg barriers (30a, 30b) of the chassis (10), extend preferably along the longitudinal edges (16,17) of the chassis, between the front belt (40a) and back belt (40b) passing through the crotch portion. The standing gathers (4, 5), the outer leg barriers (30a, 30b), or both, can optionally intersect the front belt (40a), the back belt (40b), or both. During use, the elasticized outer leg barriers (30a, 30b) can form a gasket about the legs of the user to control or prevent lateral liquid leakage.

While the pants (1000) are described particularly with reference to pants having a front and back belt, it will be understood that the chassis (10) could be incorporated into a pant (1000) that has elasticized side panels. For example, a pair of back elasticized side panels could be joined to the longitudinal side edges (16, 17) of the chassis (10) at the back waist portion, extending laterally outward therefrom. And a pair of frontal elasticized side panels could be joined to longitudinal side edges (16,17) the chassis (10) in the front waist portion, extending laterally outward therefrom. The respective front and back elasticized side panels can be coupled (e.g., with a side seam or corresponding fasteners) to form a closed pant product. In this embodiment, the elastic members (8a) of the elasticized outer leg barriers (30a, 30b) of the chassis (10) extend over the crotch portion (12) and between the front and back elasticized panels.

In yet another aspect, an absorbent article can include a re-usable outer cover that is coupled with the chassis (10) (e.g., as a disposable absorbent "insert"). The absorbent article may be adapted to be worn about the lower torso of a user similar to a diaper or pant, described herein. The re-usable outer cover may be adapted to permit insertion/removal of an absorbent chassis (10), without damaging or destroying any necessary re-usable cover components to provide the substantial like-new functionality of the re-usable cover. The standing leg gathers and outer leg barriers help to contain the bodily exudates on the chassis, and prevent them from soiling the outer cover during use. In this embodiment (not shown), the absorbent insert (10), the re-usable outer cover, or both can include fastening means to help position and removably fasten the absorbent chassis to the re-usable outer cover during use. For example, the chassis, the outer cover, or both may include fastening elements such as adhesive, hook-loop fastening system buttons, snaps, pockets, slings, straps, flaps, or any other suitable fastening systems.

### WAIST BARRIER(S)

In a preferred embodiment, exemplified in FIG. 3-4, the absorbent article (100, 1000) comprises at least one waist barrier (70) which is positioned in the front and/or back waist belt of the absorbent article and/or is positioned in the front and/or back waist region of the disposable absorbent chassis (10). Preferably, the at least one waist barrier (70) comprises a single layer of web material which is folded along a first fold line (F1) to form a waist barrier with at least 2 layers comprising an inner layer (I) and an outer layer (O).

In a preferred embodiment, as exemplified by FIG. 2A, each of the waist barriers (70) is folded along a second fold line (F2) to form a folded section (22) comprising at least 4 layers and a non-folded section (23) comprising at least 2 layers.

In an embodiment, as exemplified in FIG. 2B, the folded section (22) comprises a first section (24) comprising at least 2 layers and a second section (25) comprising at least 2 layers in which the garment-facing side of the first section (24) is bonded to the user-facing side of the second section (25) at a folded section bond site (FB). Preferably, at least one elastic element (8b) is bonded to the garment-facing side of the first section (24) and/or the user-facing side of the second section (25) between the folded section bond site (FB) and the second fold line (F2). Advantageously, such configuration allows for 2 layers of nonwoven to be present between the at least one elastic element and the skin of the wearer. This provides for improved skin comfort and less risk of indentation and irritation of the skin due to the at least one elastic elements. Even more preferably, each waist barrier (70) comprises at least 2 elastic elements. The elastic elements may be flat elastics. Advantageously, the use of flat elastics provides improved skin comfort.

In an embodiment, as exemplified in FIG. 2C, the folded section (22) comprises a first section (24) comprising at least 2 layers and a second section (25) comprising at least 2 layers in which the user-facing side of the first section (24) is bonded to the garment-facing side of the second section (25) at a folded section bond site (FB). Preferably, at least one elastic element (8b) is bonded to the user-facing side of the first section (24) and/or to the garment-facing side of the second section (25) between the folded section bond site (FB) and the second fold line (F2). Advantageously, such configuration allows for 2 layers of nonwoven to be present between the at least one elastic element and the skin of the wearer. This provides for improved skin comfort and less risk of indentation and irritation of the skin due to the at least one elastic elements. Even more preferably, each waist barrier (70) comprises at least 2 elastic elements. The elastic elements may be flat elastics. Advantageously, the use of flat elastics provides for improved skin comfort.

In an embodiment, as exemplified in FIG. 2B-2C, the user-facing side of the outer layer (O) of the non-folded section (23) is attached to the user-facing side of the liquid impervious film layer (3a) at a first bond site (B1) and wherein the user-facing side of the inner layer (I) of the non-folded section (23) is bonded to the user-facing side of the liquid-pervious topsheet (1) at a second bond site (B2).

In a preferred embodiment, as exemplified in Fig. 2B-2C, each waist barrier (70) has an inner layer (I), an outer layer (O), a distal end (7a, 7b), a first proximal position (6a, 6b), a second proximal position (6a', 6b'), a first bond site (B1), a second bond site (B2), a lifted position and a flat position, and a cross-sectional area that is a generally trilateral shape in the lifted position. The cross-sectional area is defined by the distal end (7a, 7b), the inner layer (I), the outer layer (O), a first proximal position (6a, 6b), a second proximal position (6a', 6b'), the first bond site (B1) and the second bond site (B2). Advantageously, this trilateral shaped, cross-sectional area provides for better guidance of exudates towards the point at which the inner layer and outer layer of the waist barrier have the highest separation, compared to quadrilateral shaped, cross sectional areas or conventional longitudinal standing gathers without separation between inner and outer layer. This point of highest separation provides for the best barrier function as the exudates needs to go through 2 separated layers in between which an air gap is present. This air gap in between the inner layer and the outer layer of the longitudinal standing gathers could serve as temporary storage for exudates penetrated through the inner layer and could allow this liquid to penetrate the topsheet and flow back to the absorbent core material.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which can require independent confirmation.

It is assumed that the present disclosure is not restricted to any form of embodiment described above and that some modifications can be made to the presented embodiments of the invention without departing from the scope of the embodiments as set forth in the claims that follow.

### TEST METHODS

### Emtec Tissue Softness Test Method

Softness (TS7), smoothness (TS750), and stiffness (D) properties of different sheet materials may be analyzed using an Emtec Tissue Softness Analyzer ("Emtec TSA"), available from Emtec Electronic GmbH, Leipzig, Germany, interfaced with a computer running Emtec TSA software. The Emtec TSA uses acoustic waves and has demonstrated to correlate well with hand panel tests for thin materials like tissue or nonwoven. The Emtec Tissue Softness test method may be used for determining softness, smoothness of a sheet material such as the topsheet, backsheet, standing gather and outer leg barriers as described herein.

The Emtec TSA includes a rotor with vertical blades which rotate on the test sample at a defined and calibrated rotational speed (set by manufacturer) and contact force of 100 mN. Contact between the vertical blades and the test sample creates vibrations both in the blades and in the test piece, and the resulting sound is recorded by a microphone within the instrument. The recorded sound file is then analyzed by the Emtec TSA software to determine TS7 and TS750 values. The D value is a measure of sample stiffness and is based on the vertical distance required for the contact force of the blades on test sample to be increased from 100 mN to 600 mN.

All measurements and calibrations shall be performed at standard climatic conditions of 23° C (±1° C) and 50% r.H. (±5%) in general following ISO DIN EN 20187.

The TSA is equipped with a balance for determination of grammage and thickness (gravimetric) of the samples in accordance with ISO 12625-6:2005.

### Sample preparation:

A test sample is prepared by cutting a square or circular portion of substrate/structure of interest from the absorbent article (e.g., outer cover, wrapper element, and/or standing leg gather). It is preferable that freeze spray is not used to remove the portion of the substrate to be analyzed, though it is acceptable to use freeze spray in a distal region to aid in initiating the separation of layers. Test samples are cut to a length and width (diameter in the case of a circular sample) of no less than about 90 mm and no greater than about 120 mm to ensure the sample can be clamped into the TSA instrument properly. (If an absorbent article does not contain a sufficiently large area of the substrate of interest to extract a sample of the size specified above, it is acceptable to sample equivalent material from roll stock.) Test samples are selected to avoid unusually large creases or folds within the testing region. Six substantially similar replicate samples are prepared for testing.

All samples are equilibrated at TAPPI standard temperature and relative humidity conditions (23° C.±2 C.° and 50%±2%) for at least 2 hours prior to conducting the TSA testing, which is also conducted under TAPPI conditions.

### Testing procedure:

The instrument is calibrated according to the Emtec's instructions using the 1-point calibration method with the appropriate reference standards (so-called "ref.2 samples," or equivalent, available from Emtec).

A test sample is mounted in the instrument with the surface of interest facing upward, and the test is performed according to the manufacturer's instructions. The software displays values for TS7 and TS750, when the automated instrument testing routine is complete. TS7 and TS750 are each recorded to the nearest 0.01 dB V² rms. The test sample is then removed from the instrument and discarded. This testing procedure is performed individually on the corresponding surfaces of interest of each of the six of the replicate samples (user-facing surface for standing leg gather samples and garment-facing surface for outer cover and wrapper element material samples).

The value of TS7 and, TS750 are each averaged (arithmetic mean) across the six sample replicates. The average values of TS7 and TS750 are reported to the nearest 0.01 dB V² rms.

## Claims

1. A disposable absorbent chassis () having a longitudinal direction along a longitudinal central axis (Y-Y), a transverse direction along a transverse central axis (X-X), a user-facing side and a garment-facing side, a front waist portion (13), a rear waist portion (11) and a crotch portion (12) disposed longitudinally between the front waist portion (13) and the rear waist portion (11); a first longitudinal side edge (16) and a second longitudinal side edge (17),
said disposable absorbent chassis (10) comprising a liquid-pervious topsheet (1), a backsheet (3), a liquid absorbent core (2) disposed between the liquid-pervious topsheet (1) and the backsheet (3)
wherein the backsheet (3) comprises a liquid-impervious film layer (3a) and optionally an outer cover layer (3b); the liquid-impervious film layer (3a) having a first longitudinal edge (303a), a second longitudinal edge (304a) and a liquid-impervious film layer width (WB) measured in the transverse direction; the outer cover layer (3b) having a first longitudinal edge (303b) and a second longitudinal edge (304b) and an outer cover width (WC) measured in the transverse direction;
said disposable absorbent chassis (10) further comprising
a pair of opposed elasticized outer leg barriers (30a, 30b) disposed on either side of the longitudinal central axis Y-Y comprising at least one elastic member (8a);
a pair of longitudinal standing gathers (4, 5) disposed on either side of the longitudinal central axis (Y-Y), wherein each longitudinal standing gather (4, 5) has a first proximal end (6a, 6b) attached to the chassis, a second proximal end attached to the chassis (6a', 6b'), a distal end (7a, 7b) extending towards the longitudinal central axis and comprises a single layer of web material wherein each standing leg gather (4, 5) has a lateral flap (50a, 50b) extending transversely outward toward one of the longitudinal side edges (16, 17) of the chassis (10);
and/or a waist barrier (70) disposed on either side of the transverse central axis (X-X), wherein each waist barrier (70) has a first proximal end (6a, 6b) attached to the chassis, a second proximal end (6a', 6b') attached to the chassis, a distal end (7a, 7b) extending towards the transverse central axis (X-X) and comprises a single layer of web material;
**characterized in that** the single layer of web material of each of the longitudinal standing gathers (4, 5) and/or waist barriers (70) is folded along a first fold line (F1) to form a standing gather (4, 5) and/or waist barrier (70) with at least 2 layers comprising an inner layer (I) and an outer layer (O), wherein each standing gather (4, 5) and/or waist barrier (70) is folded along a second fold line (F2) to form a folded section (22) comprising at least 4 layers and a non-folded section (23) comprising at least 2 layers.

2. A disposable absorbent chassis (10) according to claim 1, wherein the folded section (22) comprises a first section (24) comprising at least 2 layers and a second section (25) comprising at least 2 layers in which the garment-facing side of the first section (24) is bonded to the user-facing side of the second section (25) at a folded section bond site (FB); and/or wherein at least one elastic element (8b) is bonded to the garment-facing side of the first section (24) and/or to the user-facing side of the second section (25) between the folded section bond site (FB) and the second fold line (F2).

3. A disposable absorbent chassis (10) according to claim 1, wherein the folded section (22) comprises a first section (24) comprising at least 2 layers and a second section (25) comprising at least 2 layers in which the user-facing side of the first section (24) is bonded to the garment-facing side of the second section (25) at a folded section bond site (FB); and/or wherein at least one elastic element (8b) is bonded to the user-facing side of the first section (24) and/or the garment-facing side of the second section (25) between the folded section bond site (FB) and the second fold line (F2).

4. A disposable absorbent chassis (10) according to any of the preceding claims, wherein each longitudinal standing gather (4, 5) and/or waist barrier (70) comprises at least 2 elastic elements, preferably flat elastics.

5. A disposable absorbent chassis (10) according to any of the preceding claims, wherein the user-facing side of the outer layer (O) of the non-folded section (23) is attached to the user-facing side of the liquid impervious film layer (3a) at a first bond site (B1) and/or wherein the user-facing side of the inner layer (I) of the non-folded section (23) is bonded to the user-facing side of the liquid-pervious topsheet (1) at a second bond site (B2).

6. A disposable absorbent chassis (10) according to any of the preceding claims, wherein the longitudinal standing gather (4, 5) and/or waist barrier (70) has an inner layer (I), an outer layer (O), a distal end (7a, 7b), a first proximal end (6a, 6b), a second proximal end (6a', 6b'), a first bond site (B1), a second bond site (B2), a lifted position and a flat position, and a cross-sectional area that is a generally trilateral shape in the lifted position, the cross-sectional area being defined by the distal end (7a, 7b), the first proximal end (6a, 6b), the second proximal end (6a', 6b'), the inner layer (I), the outer layer (O), the first bond site (B1) and the second bond site (B2).

7. A disposable absorbent chassis (10) according to any of the preceding claims, wherein each of the elasticized outer leg barriers (30, 30b) comprises a substrate folded about a third fold line (F3) towards the longitudinal central axis (Y-Y) such that a folded portion (27) extends between a distal folded edge (26) and a substrate edge (28), in which the substrate edge (28) is located transversely inward from the distal folded edge (26), and wherein the folded portion (27) comprises a user-facing side and a garment-facing side, preferably wherein the substrate of the elasticized outer leg barriers (30a, 30b) comprises the single layer of web material folded towards the garment-facing side of the absorbent chassis (10); and/or wherein the substrate of the outer leg barriers (30a, 30b) is a separate wrapper layer (9a, 9b) joined to the backsheet,(3) and further comprising a first wrapper proximal edge (32) that is transversely inward from the distal folded edge (31) and a second wrapper proximal edge (32') that is transversely inward from the distal folded edge (31) such that a folded portion (27) extends between a distal folded edge (31) and a second proximal edge (32').

8. A disposable absorbent chassis (10) according to claim 7, wherein the user-facing side of the folded portion (27) is attached to the garment-facing surface of the outer cover layer (3b); and/or wherein the user-facing side of the folded portion (27) is attached to the garment-facing surface of the liquid-impervious film layer (3a); and/or wherein the garment-facing side of the folded portion (27) is attached to the user-facing side of the outer cover layer (3b).

9. A disposable absorbent chassis (10) according to any of claim 7-8, wherein the folded section (27) of the elasticized outer leg barriers (30a, 30b) has a folded section width (WF) measured in the transverse direction and wherein (WB - (2 x WF)) ≤ WC < WB.

10. A disposable absorbent chassis (10) any of the preceding claims, wherein the at least one elastic member (8a) is disposed within the folded portion (27) at the distal folded edge (26, 31) of the elasticized outer leg barrier (30a, 30b).

11. A disposable absorbent chassis (10) of any of the preceding claims, wherein at least 2 elastic members (8a) are disposed within the folded portion (27) at the distal folded edge (26, 31) of the elasticized outer leg barrier (30a, 30b).

12. A disposable absorbent chassis (10) according to any of the preceding claims,
wherein substrate of the elasticized outer leg barriers (30a, 30b) has one or both of: a TS7 value between 2 dB V² rms and 8 dB V² rms measured according with the Emtec test method; and a TS750 value between 3 dB V² rms and 20 dB V² rms measured according with the Emtec test method; and/or wherein the lateral flaps () of the elasticized longitudinal standing gathers (), has one or both of: a TS7 value between 2 dB V² rms and 8 dB V² rms measured according with the Emtec Tissue Softness test method; and a TS750 value between 3 dB V² rms and 20 dB V² rms measured according with the Emtec Tissue Softness test method.

13. A disposable absorbent chassis (10) according to any of the preceding claims, wherein the liquid-pervious topsheet (1) comprises a zone-treated nonwoven, partially treated to be hydrophilic.

14. A disposable absorbent article (100, 1000), selected from one of an absorbent diaper (100) or an absorbent pant (1000), including the disposable absorbent chassis (10) of any of the preceding claims; wherein the disposable absorbent article (100) comprises a pair of opposed back ear panels (20a, 20b) joined to the longitudinal side edges (16, 17) of the chassis (10) at the back waist portion (13) and a pair of opposed frontal ears (60a, 60b) joined to the longitudinal side edges (16, 17)) of the chassis (10) in the front portion (11); or wherein the disposable absorbent article (1000) comprises a front elasticized belt (40a) attached and being adjacent to the front waist portion (11) of chassis (10), and a back elasticized belt (40b) attached and being adjacent to the rear waist portion (13) of chassis (10); **characterized in that**, the elastic members (8a) of the elasticized outer leg barriers (30a, 30b), extend between the back ear panels (20a, 20b) and the front ears (60a, 60b), or the elastic members (8a) of the elasticized outer leg barriers (30a, 30b), extend between the front elasticized belt (40a) and the back elasticized belt (40b).

15. An absorbent article comprising a re-usable outer cover and a disposable absorbent Chassis (10) of claims 1-13.
